# FASCICULE DE BREVET EUROPEEN

(11) **EP 1 631 394 B1**
(45) Date de publication et mention de la délivrance du brevet: **21.03.2007**
(21) Numéro de dépôt: 04742711.7
(22) Date de dépôt: 12.05.2004
(51) Int. Cl.: B08B 3/00

(54) **PROCEDE ET INSTALLATION POUR LE NETTOYAGE DE PIECES SOUILLEES PAR DE LA MATIERE ORGANIQUE**
VERFAHREN UND EINHEIT ZUR REINIGUNG VON MIT ORGANISCHEM MATERIAL VERUNREINIGTEN TEILEN
METHOD AND UNIT FOR CLEANING PIECES CONTAMINATED WITH ORGANIC MATTER

(30) Priorité: 16.05.2003 FR 0305864
(43) Date de publication de la demande: 08.03.2006
(73) Titulaire: Clean 3 Bio System, 56000 Vannes (FR)
(72) Inventeur: DROGUE, Henri, F-56250 Trefflean (FR); GOIBIER, Martin, F-56860 Sene (FR); AUGERI, Salvatore, F-56000 Vannes (FR); GARCIA, Thierry, F-78120 Rambouillet (FR)
(74) Mandataire: Laget, Jean-Loup
(86) Numéro de dépôt international: PCT/FR2004/001155
(87) Numéro de publication internationale: WO 2004/103584

(56) Documents cités:
- WO-A-96/11072
- DE-A- 4 209 052
- US-A- 5 254 246

## Description

La présente invention concerne un procédé et une installation pour le nettoyage, en particulier le dégraissage ou le déshuilage, de pièces souillées par de la matière organique et de bio-dépollution.

Le document EP-A-0 748 518 (WO96/11072A) décrit un procédé selon le préambule de la revendication 1 et une installation selon le préambule de la revendication 2.

Le traitement par bactéries de fluides dans le domaine du traitement des eaux usées est bien connu comme l'illustre, à titre d'arrière-plan technologique, le brevet US-A-5.245.246.

Il existe depuis de nombreuses années des systèmes de nettoyage pour nettoyer des objets contaminés par des résidus organiques. Pendant longtemps, il a été utilisé comme fluide de nettoyage des solvants halogénés ou des solvants constitués par des essences minérales qui nécessitaient de prendre un grand nombre de précautions pour leur manipulation ou leur élimination, ces opérations s'avérant coûteuses.

Pour limiter les risques liés à l'utilisation de tels solvants, dont les plus fréquents étaient constitués par les risques d'incendies, de pollution, de dermatoses et de maladies respiratoires, il a été proposé le nettoyage de telles pièces au moyen d'un fluide de nettoyage apte d'une part à se charger en matière organique au contact des pièces et d'autre part à s'épurer au moyen de micro-organismes aptes à se nourrir des matières organiques contaminantes contenues dans le fluide de nettoyage. De telles installations ont notamment été décrites dans les brevets DE-4.209.052 et EP-A-0.784.518. Si une telle solution résout les inconvénients mentionnés ci-dessus lors de l'utilisation de solvants halogénés ou non halogénés, elle engendre d'autres risques, en particulier du fait de la présence de micro-organismes. Il ne peut être exclu, en effet, qu'un opérateur devant manipuler un flux de fluide de nettoyage et les pièces en vue de leur nettoyage soit placé au contact des micro-organismes contenus dans le fluide de nettoyage. Or, la présence dans ce fluide de micro-organismes pathogènes apportés par les pièces ou les salissures ne peut être totalement exclue. Ces micro-organismes étrangers risquent de provoquer chez l'opérateur des maladies de type infection ou allergie plus ou moins graves.

Un but de la présente invention est donc de proposer un procédé et une installation du type précité dont les caractéristiques permettent d'écarter ou de réduire, notamment au cours de l'opération de nettoyage des pièces, les risques de contamination d'un opérateur liés à la mise en présence de l'opérateur avec les micro-organismes destinés à épurer le fluide de nettoyage ou avec d'autres micro-organismes apportés par les pièces ou les salissures.

A cet effet, l'invention a pour objet un procédé pour le nettoyage, en particulier le dégraissage ou le déshuilage, de pièces souillées par de la matière organique, au moyen d'un fluide de nettoyage dont au moins une partie circule en boucle entre une unité de lavage des pièces dans laquelle le fluide de nettoyage se charge en matières organiques au contact des pièces et une unité de traitement dans laquelle des micro-organismes vivants dégradent biologiquement la matière organique contenue dans le fluide issu de l'unité de lavage, procédé caractérisé en ce qu'il consiste à soumettre au moins une partie du fluide de nettoyage circulant dans ladite installation à une stérilisation au moins partielle en vue de limiter, voire de supprimer, la présence de micro-organismes vivants dans le fluide de nettoyage servant dans l'unité de lavage.

L'invention a encore pour objet une installation pour le nettoyage, en particulier le dégraissage ou le déshuilage, de pièces souillées par de la matière organique au moyen d'un fluide de nettoyage dont au moins une partie circule en boucle entre une unité de lavage des pièces dans laquelle le fluide de nettoyage se charge en matières organiques au contact des pièces et une unité de traitement dans laquelle les micro-organismes vivants dégradent biologiquement la matière organique contenue dans le fluide issu de l'unité de lavage, caractérisée en ce que l'installation comporte en outre, sur le circuit de circulation de fluide, des moyens de stérilisation au moins partielle des micro-organismes contenus dans au moins une partie du fluide de nettoyage.

Grâce à la stérilisation partielle ou totale du fluide de nettoyage circulant à l'intérieur de l'installation, il est possible de limiter, voire de supprimer tout contact des mains d'un opérateur avec des micro-organismes vivants lors de l'opération de nettoyage des pièces de manière à supprimer tout risque d'infection ou d'allergies lié au contact avec les micro-organismes présents dans le fluide de nettoyage.

L'invention sera bien comprise à la lecture de la description suivante d'exemples de réalisation, en référence aux dessins annexés dans lesquels :
la figure 1 représente une vue schématique d'ensemble d'une installation conforme à l'invention et
les figures 2 à 9 représentent des vues schématiques partielles de divers modes de réalisation d'une installation conforme à l'invention.

Comme mentionné ci-dessus, l'installation, objet de l'invention, est plus particulièrement destinée à permettre le nettoyage, en particulier le dégraissage ou le déshuilage, de pièces souillées par de la matière organique et la bio-dépollution du fluide. Une telle installation est en particulier destinée au nettoyage de pièces mécaniques ou autres. Cette installation comporte, de manière en soi connue, une unité 1 de lavage des pièces dans laquelle le fluide de nettoyage se charge en matières organiques au contact des pièces et une unité 2 de traitement dans laquelle des micro-organismes vivants dégradent biologiquement la matière organique contenue dans le fluide issu de l'unité 1 de lavage. Il est bien évident que par pièce, on entend tout type d'objet.

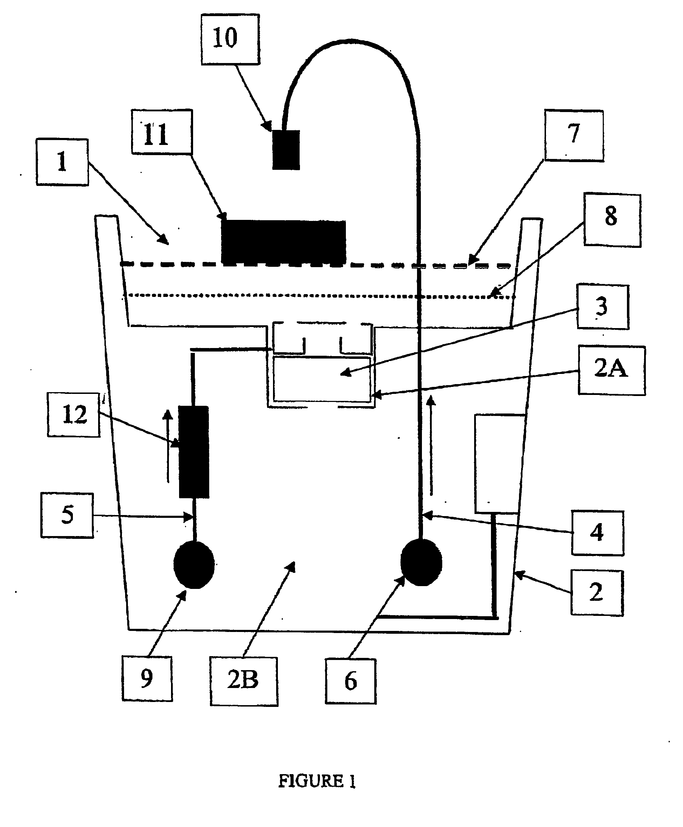

Au moins une partie du fluide de nettoyage est ainsi amenée à circuler en boucle ou en circuit fermé entre l'unité 1 de lavage et l'unité 2 de traitement. Par circuit en boucle, on entend un circuit qui permet une circulation d'au moins une partie du fluide de l'unité 1 de lavage vers l'unité 2 de traitement d'une part et de l'unité 2 vers l'unité 1 d'autre part pour permettre de ré-acheminer au moins une partie du fluide de l'unité 2 vers l'unité 1. En effet, une fois décontaminé au moins partiellement en matière organique par les micro-organismes, le fluide de nettoyage peut être réutilisé pour l'opération de lavage. L'opération de lavage peut s'effectuer sous forme d'un bain à l'intérieur de l'unité 1 de lavage ou par projection de fluide de nettoyage à la surface des pièces. C'est ce second cas qui a été choisi dans le mode de réalisation représenté à la figure 1.

De manière caractéristique à l'invention, cette installation comporte en outre, sur le circuit de circulation de fluide, des moyens 12 de stérilisation au moins partielle des micro-organismes contenus dans au moins une partie du fluide de nettoyage.

Bien évidemment, une telle installation peut affecter un grand nombre de formes et il ne sera décrit ci-après que quelques modes de réalisation de l'invention. La stérilisation au moins partielle du fluide de nettoyage permet de limiter, voire de supprimer tout contact entre les micro-organismes contenus dans le fluide de nettoyage et les mains de l'opérateur. Ainsi, le risque de contamination, d'infection ou d'allergie de l'opérateur est réduit.

Ces moyens 12 de stérilisation du fluide de nettoyage sont de préférence des moyens de stérilisation par voie physique aptes à produire de la chaleur et/ou des radiations et/ou des ultraviolets, et/ou par voie chimique en vue d'une action bactéricide et/ou bactériostatique sur les micro-organismes contenus dans le fluide de nettoyage. Ainsi, ces moyens de stérilisation peuvent être constitués par une lampe UV placée sur le circuit de circulation de fluide, en particulier sur une conduite 4 de canalisation du fluide qui s'étend par exemple dans la zone de liaison entre l'unité 1 de lavage et l'unité 2 de traitement, cette zone de liaison permettant une circulation du fluide de nettoyage depuis l'unité de traitement en direction de l'unité 1 de lavage. Ainsi, dans ce cas, les moyens 12 de stérilisation s'étendent, sur le circuit de circulation de fluide, en aval de l'unité 2 de traitement. Bien évidemment, ces moyens 12 de stérilisation peuvent encore être positionnés en d'autres emplacements de l'installation comme cela sera décrit ci-après. Ces moyens 12 de stérilisation peuvent être également constitués par des moyens de chauffage du fluide en vue de provoquer une destruction des micro-organismes. Dans ce cas, le chauffage est de préférence suivi d'un refroidissement du fluide pour dissiper les calories excédentaires et maintenir le fluide à la température normale d'utilisation entre 20 et 40°C. Enfin, ces moyens de stérilisation peuvent également être constitués par des moyens d'émission de radiations, en particulier d'ultraviolet, engendrant la destruction des micro-organismes. La stérilisation peut également s'opérer par voie chimique à l'aide d'agents chimiques tels que l'ozone. Cette stérilisation peut être totale ou partielle. On parlera de stérilisation partielle ou maîtrisée lorsque le nombre de micro-organismes, après traitement par stérilisation du fluide, est inférieur à un niveau prédéterminé mais supérieur à zéro. Le nombre de micro-organismes est dans ce cas maintenu inférieur à un seuil défini. On parlera de stérilisation totale ou complète lorsqu'on constate, après analyse biologique, une absence de micro-organismes à partir d'un échantillon de fluide de nettoyage ayant été soumis à cette étape de stérilisation. Par ailleurs, cette stérilisation peut avoir une action bactériostatique, c'est-à-dire simplement empêcher la croissance des micro-organismes présents dans le fluide, ou une action bactéricide, c'est-à-dire détruire en outre les micro-organismes présents dans le fluide.

Bien qu'un grand nombre de modes de réalisation d'une telle installation puissent être retenus, il sera décrit ci-après plus particulièrement deux modes de réalisation de l'invention. Dans les modes de réalisation décrits ci-après, l'unité 2 de traitement traversée par le fluide de nettoyage issu de l'unité 1 de lavage comporte au moins une chambre de traitement, représentée en 2A aux figures, remplie d'une matière 3 filtrante sur laquelle les micro-organismes sont immobilisés et à travers laquelle le fluide de nettoyage circule généralement de façon continue pour assurer une oxygénation des micro-organismes nécessaire à leur développement. Dans l'exemple représenté, l'unité 1 de lavage affecte la forme d'un évier à l'intérieur duquel les pièces 11 à traiter sont posées sur un caillebotis 7 reposant lui-même sur une grille 8 s'étendant au-dessus d'un orifice d'évacuation de l'unité 1 de lavage. Cette sortie d'évacuation de fluide, positionnée dans le fond du bac ou évier, est conformée de manière à ménager, à l'intérieur de cette dernière, d'une part un siphon, d'autre part la chambre 2A de traitement de l'unité de traitement constituant un bioréacteur. Ainsi, cet orifice d'évacuation délimite une chambre à parois généralement ajourées, intégrant la matière 3 filtrante sur laquelle les micro-organismes sont immobilisés. A titre d'exemple, cette matière filtrante peut être constituée de tissus tissés ou non tissés, géotextiles, fibres, céramique, terre cuite, argile, média en plastique ou en verre, mousses alvéolées, pouzzolane, lithotamne, coke métallurgique, cailloux siliceux.

Les micro-organismes immobilisés sur ce support peuvent quant à eux être constitués des genres Achronobacter, Acinetobacter, Actinomyces, Alcaligenes, Bacillus, Flavobacterium, Klebsiella, Nocardia, Pseudomonas, Streptomyces, Vibrio, Xanthomyces, Aspergillus ou autres micro-organismes pouvant participer à la biodégradation des hydrocarbures.

Cette matière 3 filtrante contient en outre des éléments nutritifs pour les micro-organismes. Ces éléments nutritifs sont de préférence constitués par des sources autres que des sources carbonées et sont de préférence encore insolubles, ou faiblement solubles, dans le fluide de nettoyage. Cette solution constructive favorise l'immobilisation des micro-organismes sur leur support de telle sorte que très peu de micro-organismes sont amenés à se détacher du support 3 et à circuler dans le fluide. Les sources nutritives peuvent être constituées de phosphore, d'azote, d'oxygène, de soufre, de magnésium, de potassium, de calcium, de fer, de manganèse et d'autres oligo-éléments.

Dans les exemples représentés aux figures 1 à 9, la chambre 2A de traitement de l'unité 2 de traitement, à l'intérieur de laquelle le fluide de nettoyage aqueux est traité par contact avec des micro-organismes, communique avec une chambre 2B de récupération et de stockage du fluide au moins partiellement épuré issu de la chambre 2A de traitement. Cette chambre 2B ou 2B2 de récupération de fluide traité peut être compartimentée, les compartiments communiquant entre eux par trop-plein. Cette chambre 2B, 2B2 de récupération de fluide traité est équipée d'une part d'un circuit 5 de recirculation de fluide vers la chambre 2A de traitement, d'autre part de moyens 4 de liaison directe ou indirecte avec l'unité 1 de lavage en vue d'une circulation du fluide en direction de l'unité 1 de lavage. Ces moyens 4 de liaison ou boucle 5 de circulation sont par exemple constitués d'une conduite de circulation de fluide à l'intérieur de laquelle le fluide est entraîné en déplacement par l'intermédiaire d'une pompe.

Ainsi, dans les exemples des figures 1 à 9, la boucle 5 de circulation de fluide entre la chambre 2B ou 2B2 de récupération de fluide traité et la chambre 2A de traitement de l'unité de traitement est constituée par une pompe 9 aspirant le fluide contenu à l'intérieur de la chambre 2B ou 2B2 pour l'amener à circuler à travers une canalisation jusqu'à introduction dans la chambre 2A où il peut s'écouler à nouveau à travers le support 3 et ainsi être à nouveau au contact des micro-organismes aptes à traiter le fluide et à le décontaminer. Ce fluide retourne ensuite dans la chambre 2B ou 2B2. De la même manière, dans les figures 1 à 7, les moyens 4 de liaison, entre la chambre 2B de récupération de fluide traité de l'unité 2 de traitement et unité 1 de nettoyage, sont constitués par une canalisation représentée en 4 aux figures. Cette canalisation 4 est équipée d'une pompe 6 d'aspiration de fluide contenu dans la chambre 2B en vue d'amener ce fluide à l'intérieur de l'unité 1 de nettoyage. Ce fluide est ainsi projeté par l'intermédiaire d'une brosse 10 sur la pièce 11 à nettoyer. Le fluide de nettoyage ainsi chargé en matière organique est alors évacué de l'unité 1 de lavage vers la chambre 2A de traitement de l'unité de traitement et un nouveau cycle peut commencer. Les pompes 6 et 9 utilisées sont, de préférence, des pompes immergées à turbine. Cette turbine est logée à l'intérieur d'un stator apte à créer un champ magnétique coopérant avec l'axe aimanté à lubrification hydrodynamique de rotation de la turbine.

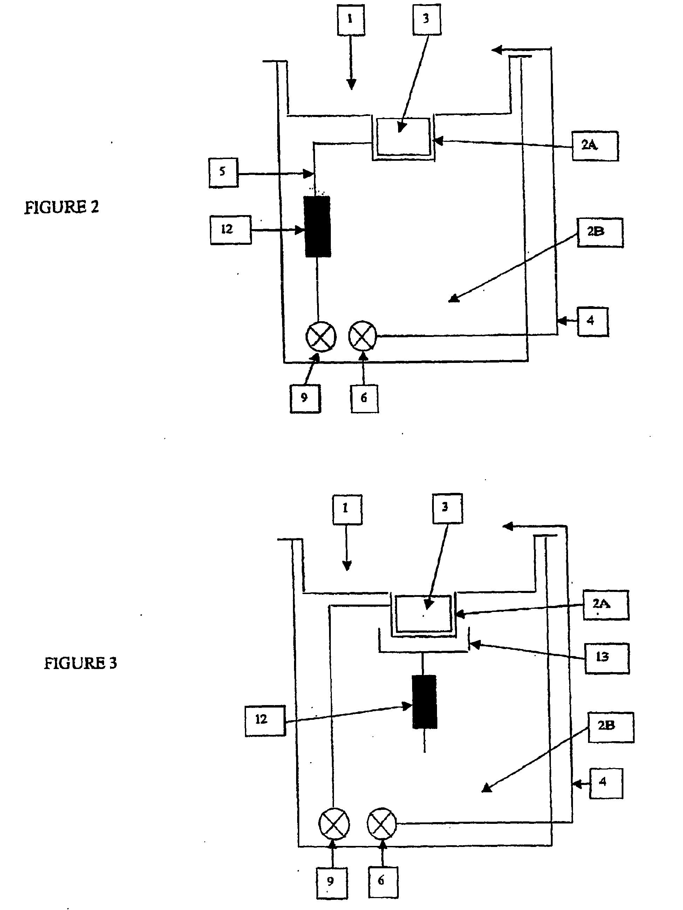

Dans l'exemple représenté aux figures 1 et 2, les moyens 12 de stérilisation sont positionnés sur le circuit 5 de re-circulation de fluide entre la première et la seconde chambre de l'unité 2 de traitement. En variante et/ou en complément, les moyens 12 de stérilisation peuvent être positionnés sur les moyens 4 de liaison entre l'unité 2 de traitement et l'unité 1 de lavage et/ou dans l'une des chambres de l'unité de traitement et/ou sur la liaison entre deux chambres de l'unité de traitement.

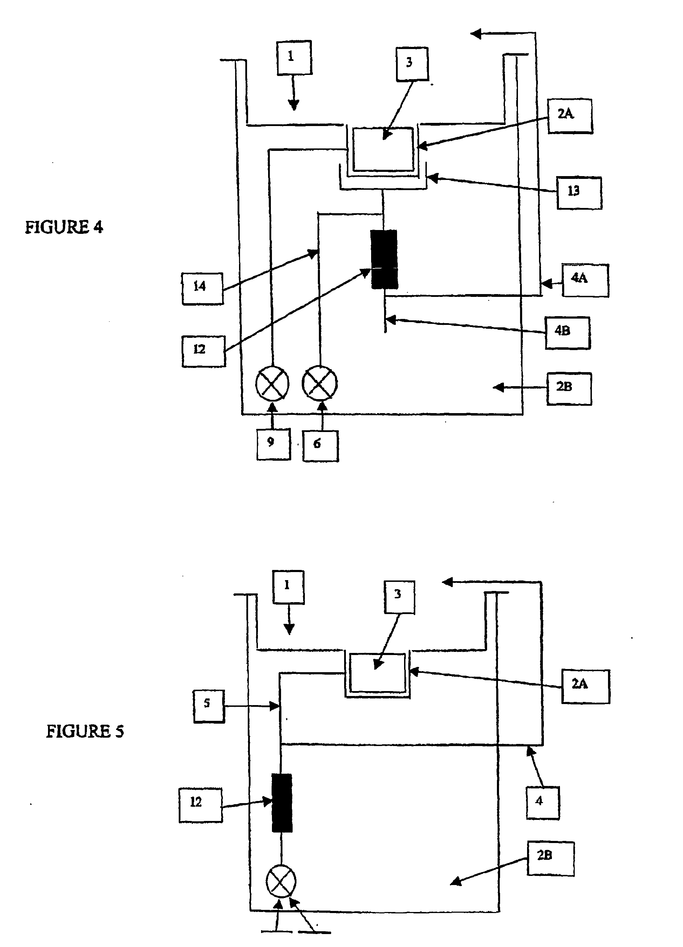

Ainsi, dans l'exemple représenté aux figures 1 et 2, le fluide pompé dans la chambre 2B par l'intermédiaire de la pompe 9 et re-circulant à travers la conduite 5 pour être amené jusqu'à la chambre 2A de l'unité 2 de traitement circule à travers une lampe à rayonnement ultraviolet 12 qui assure une stérilisation au moins partielle du fluide circulant à l'intérieur de ladite canalisation. Eu égard à la quantité de fluide re-circulant à travers cette boucle 5 de circulation, il peut être considéré que le fluide contenu dans la chambre 2B est un fluide stérile ou quasi stérile. Dans un autre mode de réalisation représenté en particulier aux figures 3 et 4, les moyens 12 de stérilisation sont positionnés entre la première et la deuxième chambre de l'unité de traitement de sorte que le fluide issu de la chambre 2A de traitement atteignant la deuxième chambre 2B est stérile. Ainsi, dans l'exemple représenté à la figure 3, il est prévu un collecteur 13 apte à canaliser le fluide de nettoyage issu de la chambre 2A de traitement, ce fluide canalisé passant à travers une canalisation équipée d'une lampe 12 à rayonnements ultraviolets pour stériliser le fluide destiné à déboucher dans la seconde chambre 2B.

Dans l'exemple représenté à la figure 4, on retrouve ce même collecteur 13 de fluide du nettoyage issu de la chambre 2A de traitement, la lampe 12 à rayonnements ultraviolets étant positionnée en sortie de ce collecteur 13 sur une conduite destinée soit par sa portion 4A à permettre une re-circulation du fluide en direction de l'unité 1 de lavage, soit par sa portion 4B à alimenter la chambre 2B de récupération de fluide traité. La pompe 6, destinée à alimenter cette conduite 4A de liaison entre chambre 2B et unité 1 de lavage, vient, par l'intermédiaire d'un piquage 14 s'étendant en amont des moyens 12 de stérilisation, alimenter cette conduite 4A. Cette disposition permet de garantir que le fluide, issu de la chambre 2B et dirigé vers l'unité 1 de lavage, a été stérilisé.

Comme l'illustre la figure 5, il est également possible de positionner les moyens de stérilisation immédiatement en aval de la pompe 6 ou 9 servant à aspirer le fluide depuis la chambre 2B de récupération de fluide traité de l'unité de traitement en direction de la chambre 2A de traitement de l'unité de traitement. Il est également prévu, en aval de ces moyens de stérilisation, un piquage permettant la liaison du contenu de la chambre 2B avec l'unité 1 de lavage. Dans ce cas, une même pompe, représentée en 6 et 9 à la figure 5, sert à la fois à l'alimentation en fluide de nettoyage de la chambre 2A et de l'unité 1 de lavage. Il convient alors de disposer, en aval des moyens de stérilisation, un sélecteur de circuit, de préférence constitué d'une vanne actionnée soit manuellement par l'opérateur, soit automatiquement selon un principe de distributeur hydraulique à clapet avec retour par ressort taré à une valeur donnée, ou une électrovanne pilotée par des instructions de commande manuelle ou automatique, alimentant au choix la chambre 2A ou l'unité 1 de lavage.

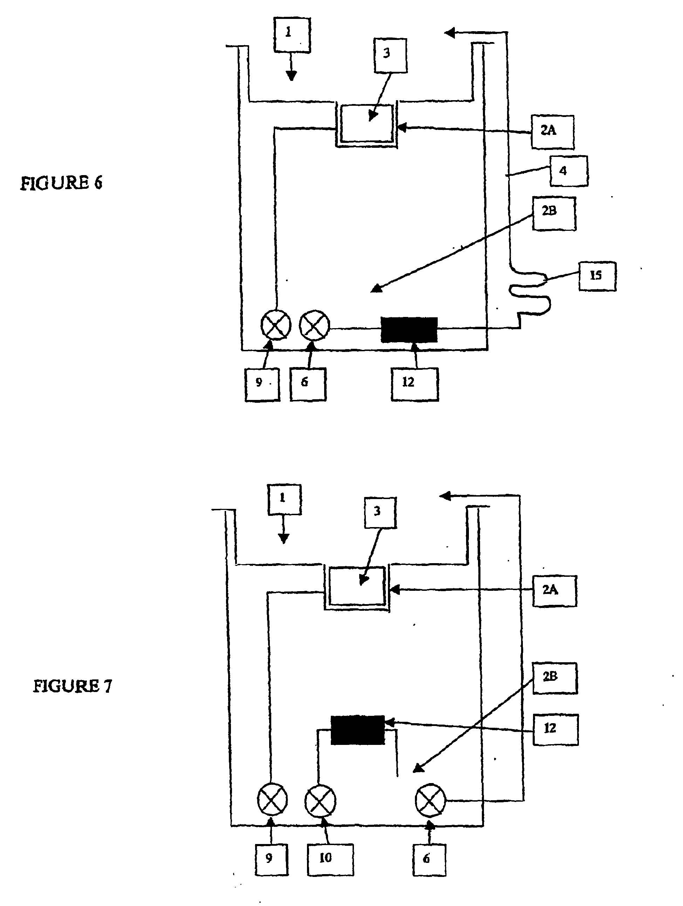

Les moyens 12 de stérilisation peuvent encore être positionnés dans la canalisation de liaison entre l'unité 2 de traitement et l'unité 1 de lavage, cette canalisation 4 de liaison permettant la circulation du fluide de nettoyage de l'unité 2 de traitement vers l'unité 1 de lavage. Cet exemple correspond à celui représenté à la figure 6 où les moyens de stérilisation sont positionnés sur la conduite 4 s'étendant entre la pompe 6, destinée à aspirer le fluide dans la chambre 2B, et l'unité 1 de lavage. Si ces moyens 12 de stérilisation sont constitués de moyens de chauffage, des moyens 15 de refroidissement leur sont de préférence associés afin de maintenir le fluide à la température normale d'utilisation entre 20 et 40°C.

Dans un autre mode de réalisation représenté à la figure 7, les moyens de stérilisation sont positionnés dans la chambre 2B de récupération de fluide traité de l'unité 2 de traitement. Ainsi, la chambre 2B est maintenue stérile ou quasi stérile par la circulation du fluide dans les moyens 12 de stérilisation au moyen d'une pompe 10 instaurant une circulation en boucle du fluide à l'intérieur de ladite chambre.

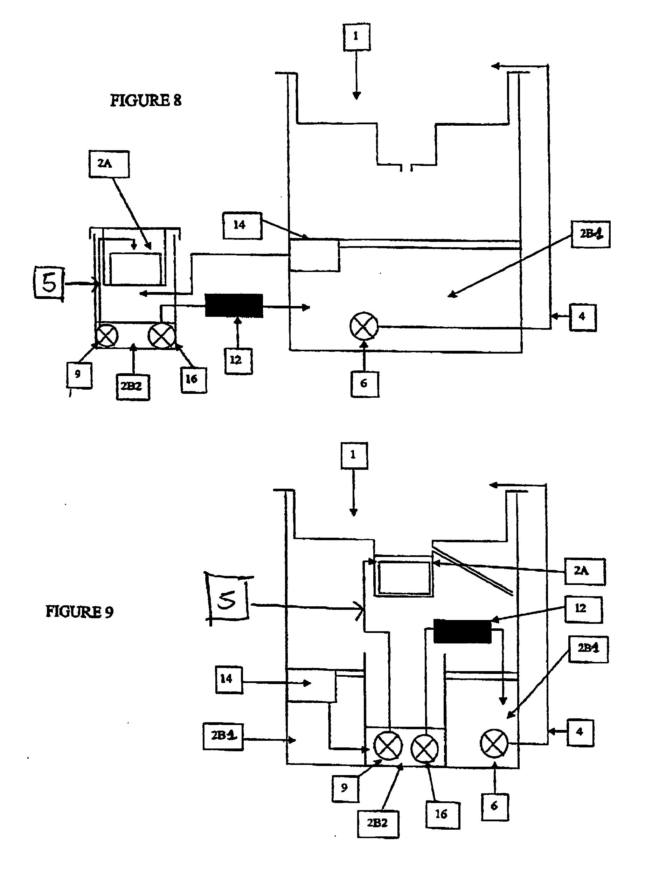

Les figures 8 et 9 illustrent deux autres modes de réalisation de l'invention dans lesquels le nombre de chambre de l'unité 2 de traitement a été augmenté. Ainsi, dans ces deux modes de réalisation, la chambre 2A de traitement de l'unité 2 de traitement, à l'intérieur de laquelle le fluide de nettoyage est traité par contact avec des micro-organismes vivants, communique avec une chambre 2B2 de récupération et de stockage du fluide issu de la chambre 2A de traitement. Cette chambre 2B2 est équipée de moyen de re-circulation de fluide vers la chambre 2A de traitement. Cette chambre 2B2 communique avec une chambre supplémentaire 2B1 constituant l'interface des chambres 2, 2A, 2B2 de l'unité 2 de traitement avec l'unité 1 de lavage. Ainsi, la chambre 2B2 est elle-même alimentée en fluide issu de l'unité 1 de lavage à partir d'une chambre 2B1. Cette chambre interface 2B1 comporte des moyens 4 de liaison avec l'unité 1 de lavage en vue d'une circulation du fluide en direction de l'unité 1 de lavage. Cette chambre 2B1 d'interface entre les autres chambres de l'unité 2 de traitement et l'unité 1 de lavage alimente au moins l'une des autres chambres de l'unité de traitement par l'intermédiaire par exemple d'un dispositif 14 de déshuilage. Ce dispositif permet d'extraire la phase légère du fluide biphasique issu de l'unité 1 de lavage. Ce dispositif comprend des moyens d'extraction de la phase légère et des moyens de commande en fonctionnement desdits moyens d'extraction. Ces moyens de commande en fonctionnement des moyens d'extraction sont constitués par exemple d'une part de deux flotteurs aptes à flotter l'un à la surface de la phase légère, l'autre à la surface de la phase lourde, d'autre part d'au moins un capteur dont l'activation autorisant la mise en fonctionnement ou respectivement l'arrêt des moyens d'extraction est asservie au positionnement relatif desdits flotteurs. Ces moyens d'extraction sont pas exemple constitués d'un conduit d'évacuation muni à son extrémité d'un flotteur maintenant le débouché de ladite extrémité de conduit dans la phase à extraire, ce conduit étant équipé d'un organe d'obturation, tel qu'une vanne commandée en ouverture et en fermeture par ledit capteur. L'organe d'obturation est commandé en ouverture lorsque la distance, séparant les deux flotteurs des moyens de commande, détectée par ledit capteur, est supérieure à une valeur prédéterminée.

Ainsi, dans l'exemple représenté à la figure 8, le fluide de nettoyage issu de l'unité 1 de lavage et chargé en matière organique s'écoule dans la chambre d'interface 2B1 qui alimente, par l'intermédiaire du dispositif de déshuilage 14, la chambre 2B2 équipée d'un circuit de re-circulation de fluide avec la chambre 2A de traitement de l'unité 2 de traitement. Ainsi, une fois le fluide traité par coopération des chambres 2B2 et 2A, le fluide est pompé par l'intermédiaire d'une pompe 16 et réacheminé dans la chambre 2B1 d'interface. Sur cette liaison entre les chambres 2B2 et 2B1, il est prévu des moyens 12 de stérilisation. Ainsi, dans ce cas, les moyens de stérilisation sont positionnés sur la liaison entre deux chambres de l'unité 2 de traitement. Le fluide est alors pompé par l'intermédiaire d'une nouvelle pompe 6 qui l'amène jusque dans l'unité 1 de lavage; De ce fait, la chambre 2B1 ne contient jamais de micro-organismes, les micro-organismes étant cantonnés aux chambres 2B2 et 2A. Dans l'exemple représenté à la figure 8, ces chambres 2B2 et 2A ont d'ailleurs été conçues de manière à être réalisées sous forme d'une unité extérieure par rapport à l'unité de lavage et à la chambre 2B1 de manière à bien séparer physiquement les éléments aptes à contenir des micro-organismes et ceux normalement exempts de micro-organismes ou en tout cas en contenant dans une quantité insuffisante pour générer une contamination.

La figure 9 illustre un autre mode de réalisation de l'invention dans lequel le fluide issu de l'unité 1 de lavage alimente la chambre 2B1 d'interface qui elle-même alimente, par un dispositif de déshuilage 14, la chambre 2B2. Cette chambre 2B2 en communication est à nouveau équipée d'un circuit de recirculation de fluide avec la chambre 2A de traitement de l'unité 2 de traitement. Une fois le fluide traité, ce fluide est ramené dans la chambre 2B1. A nouveau, des moyens 12 de stérilisation sont positionnés sur la liaison entre les chambres 2B2 et 2B1. Une pompe 6 permet, par l'intermédiaire d'une conduite 4, d'extraire le fluide de cette chambre d'interface 2B1 en vue de l'amener à l'unité 1 de lavage. Ces solutions permettent d'éviter, -de manière quasi certaine, une contamination en bactéries du fluide servant dans l'unité 1 de lavage.

Comme l'illustrent l'ensemble de ces figures, un grand nombre de modes de réalisation peuvent donc être envisagés en conservant un même objectif, à savoir limiter le nombre de micro-organismes vivants présents dans le fluide de nettoyage destiné à alimenter l'unité 1 de lavage. Ces différentes réalisations peuvent être combinées. On note que dans les exemples représentés aux figures 1 à 7, la chambre 2A de traitement de l'unité 2 de traitement est positionnée en suspension au-dessus de l'autre chambre 2B de l'unité de traitement. Cette disposition permet ainsi de maintenir le support 3 sur lequel les micro-organismes sont immobilisés dans un environnement non immergé de manière à favoriser une croissance aérobie des micro-organismes.

## Revendications

1. Procédé pour le nettoyage, en particulier le dégraissage ou le déshuilage, de pièces souillées par de la matière organique, au moyen d'un fluide de nettoyage dont au moins une partie circule en boucle entre une unité (1) de lavage des pièces dans laquelle le fluide de nettoyage se charge en matières organiques au contact des pièces et une unité (2) de traitement dans laquelle des micro-organismes vivants dégradent biologiquement la matière organique contenue dans le fluide issu de l'unité (1) de lavage,
procédé **caractérisé en ce qu'**il consiste à soumettre au moins une partie du fluide de nettoyage circulant dans ladite installation à une stérilisation au moins partielle en vue de limiter, voire de supprimer, la présence de micro-organismes vivants dans le fluide de nettoyage servant dans l'unité (1) de lavage.

2. Installation pour le nettoyage, en particulier le dégraissage ou le déshuilage, de pièces souillées par de la matière organique au moyen d'un fluide de nettoyage comprenant, une unité (1) de lavage des pièces dans laquelle le fluide de nettoyage se charge en matières organiques au contact des pièces, une unité (2) de traitement dans laquelle les micro-organismes vivants dégradent biologiquement la matière organique contenue dans le fluide issu de l'unité (1) de lavage et des moyens pour faire circuler le fluide de nettoyage en boucle entre l'unité (1) de lavage et l'unité (2) de traitement pour former ainsi un circuit de circulation de fluide,
**caractérisée en ce que** l'installation comporte en outre, sur le circuit de circulation de fluide, des moyens (12) de stérilisation au moins partielle des micro-organismes contenus dans au moins une partie du fluide de nettoyage.

3. Installation selon la revendication 2,
dans laquelle les moyens (12) de stérilisation du fluide de nettoyage sont des moyens de stérilisation par voie physique aptes à produire de la chaleur et/ou des radiations et/ou des ultraviolets et/ou par voie chimique en vue d'une action bactéricide et/ou bactériostatique sur les micro-organismes contenus dans ledit fluide de nettoyage.

4. Installation selon l'une des revendications 2 et 3,
dans laquelle l'unité (2) de traitement traversée par le fluide de nettoyage issu de l'unité (1) de lavage comporte au moins une chambre (2A) de traitement remplie d'une matière (3) filtrante sur laquelle les micro-organismes vivants sont immobilisés et à travers laquelle le fluide de nettoyage circule.

5. Installation selon la revendication 4,
dans laquelle a matière (3) filtrante contient en outre des éléments nutritifs pour les micro-organismes, ces éléments nutritifs, de préférence constitués par des sources autres que des sources carbonées, étant de préférence insolubles ou faiblement solubles dans le fluide de nettoyage.

6. Installation selon la revendication 4,
dans laquelle la chambre (2A) de traitement de l'unité (2) de traitement à l'intérieur de laquelle le fluide de nettoyage est traité par contact avec des micro-organismes vivants communique avec une chambre (2B) de récupération et de stockage du fluide issu de la chambre (2A) de traitement, cette chambre (2B) étant équipée d'une part d'un circuit (5) de re-circulation de fluide vers la chambre (2A) de traitement, d'autre part de moyens (4) de liaison avec l'unité (1) de lavage en vue d'une circulation du fluide en direction de l'unité (1) de lavage.

7. Installation selon la revendication 4,
dans laquelle la chambre (2A) de traitement de l'unité (2) de traitement à l'intérieur de laquelle le fluide de nettoyage est traité par contact avec des micro-organismes vivants communique avec une chambre (2B2) de récupération et de stockage du fluide issu de la première chambre (2A) de traitement, cette chambre (2B2) équipée d'un circuit (5) de re-circulation de fluide vers la chambre (2A) de traitement étant elle-même en communication avec une chambre (2B1) complémentaire constituant une interface des autres chambres de l'unité de traitement avec l'unité de lavage, cette chambre interface (2B1) comportant des moyens (4) de liaison avec l'unité (1) de lavage en vue d'une circulation de fluide en direction de l'unité (1) de lavage.

8. Installation selon la revendication 7,
dans laquelle la chambre (2B1) d'interface entre les autres chambres de l'unité (2) de traitement et l'unité (1) de lavage alimente en fluide les autres chambres de l'unité de traitement par l'intermédiaire d'un dispositif (14) de déshuilage.

9. Installation selon l'une des revendications 6 ou 7,
dans laquelle les moyens (12) de stérilisation sont positionnés sur la liaison entre deux chambres de l'unité de traitement, en particulier sur le circuit (5) de re-circulation de fluide entre la chambre (2A) de traitement et la chambre (2B, 2B2) de récupération et de stockage du fluide issu de la chambre (2A) de traitement et/ou sur les moyens (4) de liaison entre l'unité (2) de traitement et l'unité (1) de lavage et/ou dans l'une des chambres de l'unité (2) de traitement.

10. Installation selon la revendication 9,
dans laquelle les moyens (12) de stérilisation sont positionnés entre la chambre (2A) de traitement et la chambre (2B, 2B2) de récupération et de stockage de fluide traité de l'unité (2) de traitement de sorte que le fluide issu de la chambre (2A) de traitement atteignant l'autre chambre (2B, 2B2) est stérile.

11. Installation selon l'une des revendications 6 à 10,
dans laquelle la chambre (2A) de traitement de l'unité (2) de traitement est positionnée en suspension au-dessus de la chambre (2B, 2B2) de stockage de fluide traité de l'unité (2) de traitement.

12. Installation selon l'une des revendications 4 à 11,
dans laquelle l'unité (1) de lavage est équipée d'une sortie d'évacuation de fluide à l'intérieur de laquelle est ménagée la chambre (2A) de traitement de l'unité (2) de traitement.

13. Installation selon l'une des revendications 2 à 7,
dans laquelle l'unité (2) de traitement est équipée de moyens (4) de liaison avec l'unité (1) de lavage en vue d'une circulation du fluide en direction de l'unité (1) de lavage, les moyens (12) de stérilisation étant positionnés dans la canalisation de liaison entre lesdites unités (2, 1).

## Claims

1. A method for cleaning, in particular degreasing or deoiling, articles soiled by organic material, by means of a cleaning fluid whereof at least some circulates in a loop between a unit (1) for washing articles, in which the cleaning fluid is loaded with organic materials in contact with the articles, and a treatment unit (2), in which living micro-organisms biologically degrade the organic material in the fluid exiting the washing unit (1),
the method being **characterised in that** it consists in performing an at least partial sterilisation of at least some of the cleaning fluid circulating in the said installation in order to limit or even eliminate the presence of living micro-organisms in the cleaning fluid used in the washing unit (1).

2. An installation for cleaning, in particular degreasing or deoiling, articles soiled by organic material, by means of a cleaning fluid, comprising a unit (1) for washing articles, in which the cleaning fluid is loaded with organic materials in contact with the articles, a treatment unit (2), in which the living micro-organisms biologically degrade the organic material in the fluid exiting the washing unit (1), and means for causing the cleaning fluid to circulate in a loop between the washing unit (1) and the treatment unit (2) in order thus to form a fluid circulation circuit,
**characterised in that** the installation further has in the fluid circulation circuit means (12) for at least partial sterilisation of the micro-organisms in at least some of the cleaning fluid.

3. An installation according to Claim 2, in which the means (12) for sterilisation of the cleaning fluid are physical sterilisation means suitable for producing heat and/or radiation and/or ultraviolet rays and/or chemical sterilisation means in order to give a bactericidal and/or bacteriostatic action on the micro-organisms in the said cleaning fluid.

4. An installation according to either of Claims 2 and 3, in which the treatment unit (2) through which the cleaning fluid exiting the washing unit (1) passes has at least one treatment chamber (2A) filled with a filtering material (3) on which the living micro-organisms are immobilised and through which the cleaning fluid circulates.

5. An installation according to Claim 4, in which the filtering material (3) further contains nutritive elements for the micro-organisms, these nutritive elements, which are preferably formed by sources other than carbon-containing sources, preferably being insoluble or slightly soluble in the cleaning fluid.

6. An installation according to Claim 4, in which the treatment chamber (2A) of the treatment unit (2) inside which the cleaning fluid is treated by contact with living micro-organisms communicates with a chamber (2B) for recovery and storage of the fluid exiting the treatment chamber (2A), this chamber (2B) being equipped on the one hand with a circuit (5) for re-circulating fluid to the treatment chamber (2A) and on the other with means (4) for connection with the washing unit (1) in order to circulate fluid in the direction of the washing unit (1).

7. An installation according to Claim 4, in which the treatment chamber (2A) of the treatment unit (2) inside which the cleaning fluid is treated by contact with living micro-organisms communicates with a chamber (2B2) for recovery and storage of the fluid exiting the first treatment chamber (2A), this chamber (2B2), which is equipped with a circuit (5) for re-circulating fluid to the treatment chamber (2A), itself being in communication with a complementary chamber (2B1) forming an interface between the other chambers of the treatment unit and the washing unit, this interface chamber (2B1) having means (4) for connection with the washing unit (1) in order to circulate fluid in the direction of the washing unit (1).

8. An installation according to Claim 7, in which the chamber (2B1) for interface between the other chambers of the treatment unit (2) and the washing unit (1) supplies the other chambers of the treatment unit with fluid by way of a deoiling device (14).

9. An installation according to either of Claims 6 and 7, in which the means (12) for sterilisation are positioned at the connection between two chambers of the treatment unit, in particular on the circuit (5) for re-circulating fluid between the treatment chamber (2A) and the chamber (2B, 2B2) for recovery and storage of the fluid exiting the treatment chamber (2A) and/or on the means (4) for connection between the treatment unit (2) and the washing unit (1) and/or in one of the chambers of the treatment unit (2).

10. An installation according to Claim 9, in which the means (12) for sterilisation are positioned between the treatment chamber (2A) and the chamber (2B, 2B2) for recovery and storage of fluid treated by the treatment unit (2) such that the fluid exiting the treatment chamber (2A) and reaching the other chamber (2B, 2B2) is sterile.

11. An installation according to one of Claims 6 to 10, in which the treatment chamber (2A) of the treatment unit (2) is positioned suspended above the chamber (2B, 2B2) for storage of fluid treated by the treatment unit (2).

12. An installation according to one of Claims 4 to 11, in which the washing unit (1) is equipped with an outlet for evacuating fluid, inside which the treatment chamber (2A) of the treatment unit (2) is made.

13. An installation according to one of Claims 2 to 7, in which the treatment unit (2) is equipped with means (4) for connection with the washing unit (1) in order to circulate fluid in the direction of the washing unit (1), the means (12) for sterilisation being positioned in the connection duct between the said units (2, 1).

## Patentansprüche

1. Verfahren zur Reinigung, insbesondere zur Entfettung oder Entölung von mit organischem Material verunreinigten Teilen mithilfe einer Reinigungsflüssigkeit, von der mindestens ein Teil im geschlossenen Kreislauf zwischen einer Teilewascheinheit (1), in der die Reinigungsflüssigkeit an den Teilen anliegendes organisches Material aufnimmt, und einer Behandlungseinheit (2) umläuft, in der lebende Mikroorganismen das organische Material, das in der Flüssigkeit aus der Wascheinheit (1) enthalten ist, biologisch abbauen, **dadurch gekennzeichnet, dass** mindestens ein Teil der Reinigungsflüssigkeit, die in der genannten Anlage umläuft, zumindest teilweise sterilisiert wird, um das Vorhandensein von lebenden Mikroorganismen in der Reinigungsflüssigkeit, die der Wascheinheit (1) zugeführt wird, zu begrenzen und sogar auszuschließen.

2. Anlage für die Reinigung, insbesondere zur Entfettung oder Entölung von mit organischem Material verunreinigten Teilen mithilfe einer Reinigungsflüssigkeit, die eine Teilewascheinheit (1), in der die Reinigungsflüssigkeit an den Teilen anliegendes organisches Material aufnimmt, eine Behandlungseinheit (2), in der lebende Mikroorganismen das organische Material, das in der Flüssigkeit aus der Wascheinheit (1) enthalten ist, biologisch abbauen, und Mittel umfasst, die für den Umlauf der Reinigungsflüssigkeit im geschlossenen Kreislauf zwischen der Wascheinheit (1) und der Behandlungseinheit (2) sorgen und somit einen Flüssigkeitskreislauf bilden, **dadurch gekennzeichnet, dass** die Anlage im Flüssigkeitskreislauf zudem Sterilisationsmittel (12) umfasst, die die Mikroorganismen, die in mindestens einem Teil der Reinigungsflüssigkeit enthalten sind, zumindest teilweise abtöten.

3. Anlage nach Anspruch 2, in der die Mittel (12) zur Sterilisation der Reinigungsflüssigkeit Mittel zur Sterilisation auf physikalischem Wege durch Erzeugung von Hitze und/oder Strahlung und/oder W-Strahlen und/oder auf chemischem Wege durch bakterizide und/oder bakteriostatische Wirkung auf die in der Reinigungsflüssigkeit enthaltenen Mikroorganismen sind.

4. Anlage nach einem der Ansprüche 2 und 3, in der die Behandlungseinheit (2), durch die die Reinigungsflüssigkeit aus der Wascheinheit (1) fließt, mindestens eine Behandlungskammer (2A) umfasst, die mit einem Filtermaterial (3) gefüllt ist, an dem die lebenden Mikroorganismen aufgehalten werden und durch das die Reinigungsflüssigkeit umläuft.

5. Anlage nach Anspruch 4, in der das Filtermaterial (3) zudem Nährstoffe für die Mikroorganismen enthält, wobei die Nährstoffe, die vorzugsweise nicht aus carbonierten Quellen stammen, in der Reinigungsflüssigkeit vorzugsweise nicht oder schlecht löslich sind.

6. Anlage nach Anspruch 4, in der die Behandlungskammer (2A) der Behandlungseinheit (2), in der die Reinigungsflüssigkeit durch Kontakt mit lebenden Mikroorganismen behandelt wird, mit einer Kammer (2B) für die Rückgewinnung und Speicherung der Flüssigkeit aus der Behandlungskammer (2A) verbunden ist, wobei die Kammer (2B) einerseits mit einem Kreislauf (5) für den Rückumlauf der Flüssigkeit zur Behandlungskammer (2A) und andererseits mit Mitteln (4) zur Verbindung mit der Wascheinheit (1) ausgerüstet ist, damit die Flüssigkeit zur Wascheinheit (1) fließt.

7. Anlage nach Anspruch 4, in der die Behandlungskammer (2A) der Behandlungseinheit (2), in der die Reinigungsflüssigkeit durch Kontakt mit lebenden Mikroorganismen behandelt wird, mit einer Kammer (2B2) für die Rückgewinnung und Speicherung der Flüssigkeit aus der ersten Behandlungskammer (2A) verbunden ist, wobei die Kammer (2B2) mit einem Kreislauf (5) für den Rückumlauf der Flüssigkeit zur Behandlungskammer (2A) verbunden ist, die selbst mit einer zusätzlichen Kammer (2B1) verbunden ist, die eine Verbindungskammer zwischen den anderen Kammern der Behandlungseinheit und der Wascheinheit bildet, wobei die Verbindungskammer (2B1) Mittel (4) zur Verbindung mit der Wascheinheit (1) umfasst, damit die Flüssigkeit zur Wascheinheit (1) fließt.

8. Anlage nach Anspruch 7, in der die Verbindungskammer (2B1) zwischen den anderen Kammern der Behandlungseinheit (2) und der Wascheinheit (1) die anderen Kammern der Behandlungseinheit über eine Entölungsvorrichtung (14) mit Flüssigkeit versorgt.

9. Anlage nach einem der Ansprüche 6 oder 7, in der die Sterilisationsmittel (12) in der Verbindung zwischen zwei Kammern der Behandlungseinheit, insbesondere im Kreislauf (5) für den Rückumlauf der Flüssigkeit zwischen der Behandlungskammer (2A) und der Kammer (2B, 2B2) für die Rückgewinnung und Speicherung der Flüssigkeit aus der Behandlungskammer (2A) und/oder in den Verbindungsmitteln (4) zwischen der Behandlungseinheit (2) und der Wascheinheit (1) und/oder in einer der Kammern der Behandlungseinheit (2) angebracht sind.

10. Anlage nach Anspruch 9, in der die Sterilisationsmittel (12) zwischen der Behandlungskammer (2A) und der Kammer (2B, 2B2) für die Rückgewinnung und Speicherung der behandelten Flüssigkeit aus der Behandlungskammer (2) angebracht sind, sodass die Flüssigkeit aus der Behandlungskammer (2A), die in die andere Kammer (2B, 2B2) fließt, steril ist.

11. Anlage nach einem der Ansprüche 6 bis 10, in der die Behandlungskammer (2A) der Behandlungseinheit (2) hängend über der Kammer (2B, 2B2) für die Speicherung der behandelten Flüssigkeit aus der Behandlungseinheit (2) angebracht ist.

12. Anlage nach einem der Ansprüche 4 bis 11, in der die Wascheinheit (1) mit einem Auslauf für die Flüssigkeit ausgerüstet ist, in dem die Behandlungskammer (2A) der Behandlungseinheit (2) untergebracht ist.

13. Anlage nach einem der Ansprüche 2 bis 7, in der die Behandlungseinheit (2) mit Mitteln (4) für die Verbindung mit der Wascheinheit (1) ausgerüstet ist, damit die Flüssigkeit zur Wascheinheit (1) fließt, wobei die Sterilisationsmittel (12) in der Verbindungsleitung zwischen den Einheiten (2, 1) angebracht sind.
